# EUROPEAN PATENT SPECIFICATION

(11) **EP 1 323 450 B1**
(45) Date of publication and mention of the grant of the patent: **22.09.2004**
(21) Application number: 01310573.9
(22) Date of filing: 18.12.2001
(51) Int. Cl.: A61M 37/00, A61M 5/315

(54) **Syringe device**
Spritzvorrichtung
Seringue

(43) Date of publication of application: 02.07.2003
(73) Proprietor: Rexam Pharma GmbH, 79395 Neuenburg (DE)
(72) Inventor: Birmelin, Uwe, 79424 Auggen (DE)
(74) Representative: Maucher, Wolfgang, Dipl.-Ing.

(56) References cited:
- EP-A- 0 292 936
- EP-A- 0 564 038
- WO-A-99/53991
- DE-C- 19 734 385
- US-A- 4 846 793
- US-A- 5 370 628
- US-A- 5 558 637

## Description

The present invention relates to a syringe device as described below for subcutaneous injection of a pellet of medicament, such as those used during cancer treatment procedures. In particular, the invention provides a compact syringe device presented below by use of a telescopic/locking plunger arrangement.

According to the prior art, it is known to provide a syringe for subcutaneous injections, comprising a syringe barrel with a hollow needle attached thereto and a plunger, having a plunger rod capable of moving within the hollow needle, to drive the pellet of medicament through and out of the needle during the injection. Before the syringe is supplied to the user, one or more pellets of medicament are placed into the syringe barrel in alignment with the hollow needle. The plunger is then inserted into the barrel in its extended position, with the plunger rod taking up a position within the syringe barrel adjacent to the needle. A protective cover is then placed over the free end of the needle to protect the contents of the syringe. The plunger is maintained in this extended configuration by a locking collar, to prevent accidental activation of the syringe. Before first use, the user of the syringe removes the locking collar to enable the plunger to be depressed. The user then inserts the syringe needle under the skin as required and finally depresses the plunger. On depression of the plunger, the plunger rod drives one or more pellets of medicament through the hollow needle, to be deposited in the required location under the skin.

A disadvantage of this type of arrangement is that the syringe, primed with medicament, is relatively long and cumbersome, because of the plunger's extended configuration. Furthermore, removal of the collar before first use requires the use of two hands. Thus, an aim of the present invention is to provide a syringe for subcutaneous injection of a pellet of medicament that is compact and simple to operate.

US 5,558,637 discloses an injection device for implants where a plunger showing a plurality of projections that bend into the direction of the push button when the plunger is pushed inwards into the implant housing and which insert into an edge at the upper end of the housing when it is tried to remove the plunger from it in the opposite direction with the aim to allow for single use only of the injection device. EP 0 292 936 shows a device for administering solid or semisolid preparations to an organism subcutaneously where a guide member of a plunger snugly fitting into the barrel is connected to a terminal rod portion that allows to push implants or the like through a needle. US 5,370,628 shows a syringe for injection of liquids where the plunger is improved by having two portions which telescope into each other to provide a shortened length and if extended are in a lock-and-detent position to provide for the full length whereby the part with the pushbutton is inserted into the piston part surrounding it that is adapted tightly to the barrel to allow for pushing liquid through the terminal needle of the barrel part.

The present invention provides a syringe device comprising a barrel for holding one or more tablets of medicament, a hollow needle attached to the barrel and a plunger adapted to move within the barrel, the plunger having a plunger rod adapted to move within the hollow needle to drive a medicament tablet through the needle, characterised in that the plunger is composed of a plurality of segments, telescopically arranged one with the other, the plunger rod forming the innermost segment of the plunger, and each segment having a catch to lock the segment in its extended position wherein the catch on the plunger rod is adapted to prevent the plunger rod being removed from the barrel but allows the rod to be depressed through the barrel once the segments are fully extended and locked together.

An advantage of this telescoping/locking arrangement is that the syringe device is very compact and is wholly contained within the syringe barrel before first use, making it more robust and less likely to be damaged during transport. The total length of the syringe device (as supplied to the user) is only limited by the length of the plunger rod, which is itself limited only by the length of the hollow needle, i.e. the plunger rod has to be of sufficient length to drive the medicament tablet through the full length of the needle and then out of its free end. Furthermore, by containing the whole plunger within the syringe barrel, the device is more robust and the plunger is less likely to be damaged during transport.

The syringe device according to the invention is supplied to the user with the plunger segments in a telescopically collapsed condition i.e. lying one within another, the syringe needle attached to the syringe body and primed with medicament. On first use, the user of the syringe withdraws the plunger from the barrel and the plunger segments extend and lock together in their extended position. The plunger rod forms the innermost segment of the plunger and is locked inside the barrel by a catch. Thus, when the plunger segments are extended, the plunger rod is maintained within the barrel, aligned with the hollow needle.

The catch arrangement between the plunger rod and the syringe barrel preferably comprises a resilient projection, which engages in a slot. The projection has a catch portion which has a shallow sloping face in the direction of movement of the plunger towards the hollow needle and a steep or vertical face in the direction of movement of the plunger away from the needle, towards the outside of the barrel. Thus, as the plunger segments are extended, the plunger rod is held in the syringe barrel by the catch arrangement, because of the engagement of the steep (or vertical) engagement face of the catch portion against the edge of the slot. Once the plunger segments are fully extended, they lock together forming a rigid plunger. The user then depresses the plunger and the catch between the plunger rod and the syringe barrel disengages (because the shallow sloping face of the catch portion slides past the edge of the slot allowing the catch to be released). The plunger rod is then depressed through the syringe needle, driving a tablet of medicament in front of it. When the medicament has been delivered, the user removes the syringe needle from the injection site. The plunger rod is fully depressed and extends the full length of the hollow needle (or slightly beyond the end of it), presenting a blunt end to the syringe and thereby preventing accidental needle stick injuries.

Advantageously, the plunger rod is locked in its fully depressed position. This may be simply achieved by providing another catch at the needle end of the syringe barrel to prevent the plunger rod being withdrawn through the needle. In this arrangement, the resilient projection part of the catch arrangement is advantageously formed on the plunger rod and the slot is provided in the barrel. Thus, the second catch (to hold the plunger rod in its fully depressed position) is simply provided by a second slot, into which the resilient projection engages when the plunger is fully depressed.

Another disadvantage of such conventional syringes having hollow needles is that the user of the syringe has to be careful to ensure the medicament is retained within the syringe needle until it is inserted under the skin. The hollow needle has to be large enough to allow the pellet of medicament to pass through it, without the user having to apply excessive force to the plunger. Thus, once the syringe is primed with medicament, a user has to be careful to hold the syringe substantially horizontal, otherwise the pellets of medicament can fall through then hollow needle and out of its open end once the cover is removed.

In the present invention, this problem is overcome by the provision of a clamp, which has a first portion designed to slide inside the hollow needle and a second portion, which cannot fit inside the needle but is used to align the clamp with the needle. Preferably, the clamp is weighted to ensure that it drops into the needle and the second portion self aligns in the syringe barrel.

Pellets of medicament are inserted into the clamp, then the clamp primed with the pellets is dropped into the open end of the syringe barrel and the clamp self aligns with the hollow needle. Finally the plunger is inserted in the barrel with the plunger rod aligned with the needle. The clamp is designed to be resilient so that it can expand to allow the pellets to be inserted and thereafter resumes its original configuration, to hold the pellets firmly therein. The resilience of the clamp may be provided by the use of a flexible elastic material or alternatively, the clamp may comprise a plurality of flexible fingers.

Advantageously, the end of the clamp facing the barrel has a tapered orifice to assist in aligning the plunger rod with the pellet of the medicament within the clamp. Thus, once the syringe is primed with medicament and the plunger is inserted into the syringe barrel, the plunger rod is held rigidly between the tapered orifice in the second portion of the clamp at one end and the catch arrangement between the plunger segments at the other end.

Preferred embodiments of the invention are:

A syringe device as mentioned above, wherein the catch (6) between the plunger rod (35) and the barrel (1) comprises a resilient projection (36) adapted to engage in a slot (11) and the projections has a smooth tapered face (361) in the direction of travel of the plunger rod (35) towards the free end of the needle (2) and a steep or vertical flank (362), which engages in the slot (11) when the plunger rod (35) is being pulled in a direction away from needle (2).

A syringe device as mentioned in the last paragraph or after discussion of the prior art, wherein the plunger (3) comprises a plunger rod (35) and a body segment (31) telescopically arranged with the plunger rod (35) inside the body segment (31).

A syringe device as mentioned after discussion of the prior art wherein another catch (65) is present at the needle end of the syringe barrel (1) to prevent the plunger rod (35) from being withdrawn through the needle, allowing to lock it in its fully depressed position.

A syringe device according to any of the preceding three paragraphs or as mentioned after discussion of the prior art claims, further comprising a clamp (5) adapted to firmly hold one or more medicament tablets (10) within the syringe barrel (1) and prevent the tablet (10) from falling through the hollow needle (2).

A syringe device according to the last paragraph, wherein the clamp (5) is shaped to align the tablet with the hollow needle (2).

A syringe device according to any one of the last two paragraphs, wherein the clamp (5) is shaped to direct the plunger rod (35) towards the medicament tablet (10).

A syringe device according to any one of the last three paragraphs, wherein the clamp (5) has a first portion (51) designed to slide into the hollow needle and a second portion (55) which cannot fit inside the needle but is used to align the clamp with the needle, and the clamp is weighted to ensure that it drops into the needle and the second portion self aligns in the syringe barrel.

A syringe device according to any one of the preceding 7 paragraphs or a mentioned after the discussion of the prior art, wherein the plunger rod (35) is of sufficient length to extend the full length of the needle (2) when fully depressed.

The invention will now be described, by way of example only, with reference to the accompanying drawings, in which
FIGURE 1 shows an exploded isometric view of the syringe device according to the invention.
FIGURE 2 shows a side view of the syringe device according to the invention, as supplied to a user (before use).
FIGURE 3 shows a side view of the syringe device shown in Figure 2, after the user has extended the plunger (ready for use).
FIGURE 4 shows a side view of the syringe device shown in the previous figures with the plunger fully depressed by the user and the medicament ejected (after use).
FIGURE 5 shows an isometric view of the medicament clamp according to the invention.
FIGURE 6 shows a side section view through the syringe device shown in figure 2, as supplied to a user, i.e. with the medicament tablets inserted in the medicament clamp, the claim inserted into the syringe needle and the plunger in a telescopically collapsed configuration.
FIGURE 7 shows an enlarged view of part of figure 6, detailing the medicament clamp with the pellets inserted therein.
FIGURE 8 shows a side section view through the syringe device shown in figure 3, with the plunger fully extended.
FIGURE 9 shows an enlarged view of part of figure 8, detailing the catch arrangement between the plunder segments.

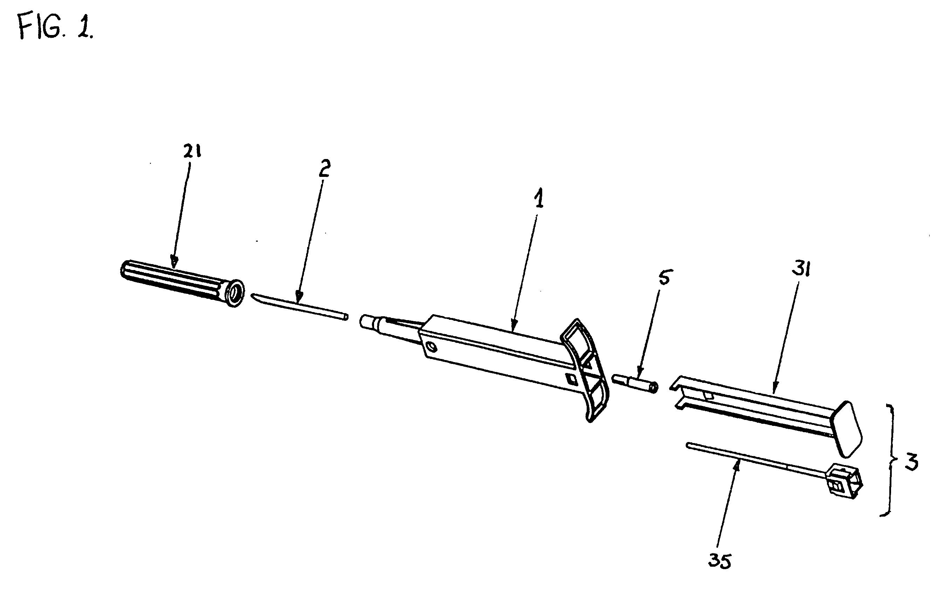

Referring to Figure 1, a syringe according to the invention generally comprises a syringe barrel 1, a hollow needle 2 and a plunger 3 comprising a plunger segment 31 and a plunger rod 35. The hollow needle 2 is protected by a removeable cover 21 and pellets of medicament are held in a medicament clamp 5.

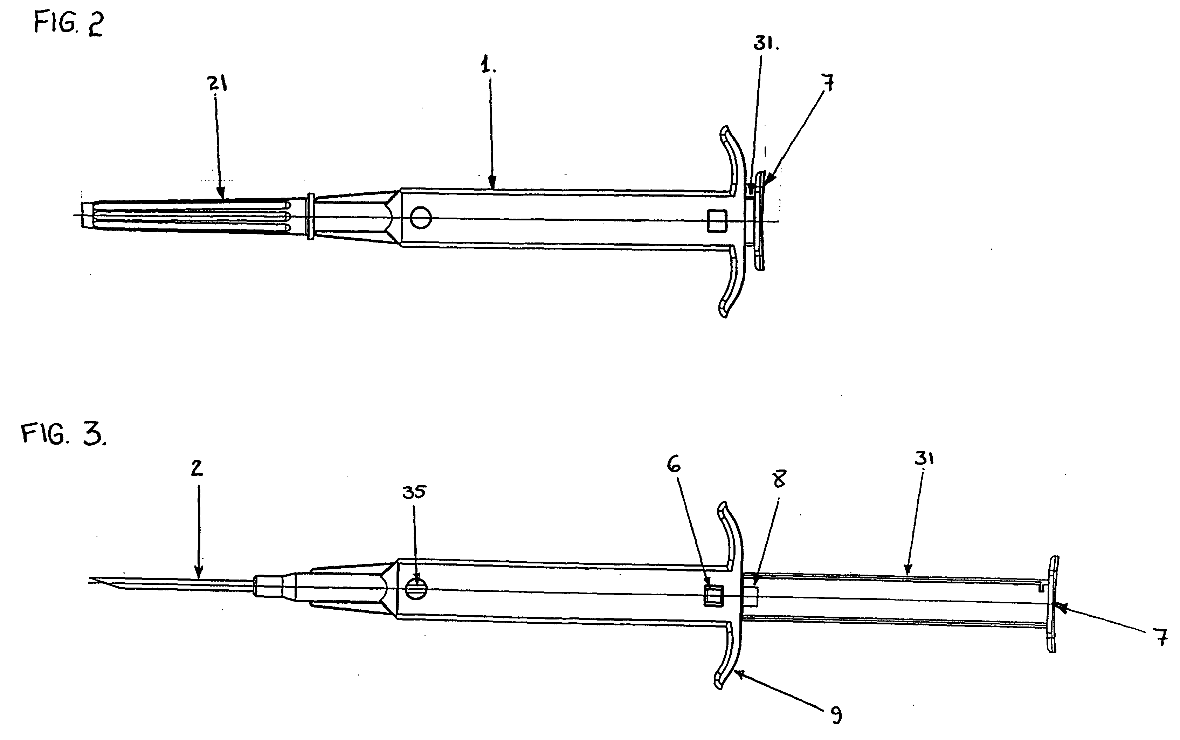

As shown in Figure 2, the syringe is supplied to the user already assembled and loaded with one or more medicament tablets. The hollow needle 2 is attached to one end of the syringe barrel 1. The plunger segment 31 and plunger rod 35 are assembled together telescopically collapsed and then inserted into the other end of the syringe barrel 1, leaving just the plunger handle 7 protruding outside the syringe barrel 1 and the protective cover 21 is placed over the needle 2.

Referring to Figure 3, a user then pulls the plunger handle 7 to extend the telescopically arranged plunger segment 31 until it locks with the plunger rod 35 in its fully open position, by means of a catch arrangement 8, to form a rigid plunger 3. Plunger rod 35 and syringe barrel 1 also have a co-operating catch arrangement 6, to prevent the plunger rod 35 being entirely removed from the barrel 1, when the plunger 3 is extended. The catch arrangement 6, between the plunger rod 35 and the syringe barrel 1, ensures that the plunger rod 35 (and therefore the rigid plunger 3) is not pulled outside the syringe barrel 1, but remains within the barrel 1 aligned with the medicament tablets therein. The syringe barrel 1 is provided with a conventional flange 9, by which the user can support the barrel 1, whilst administering an injection.

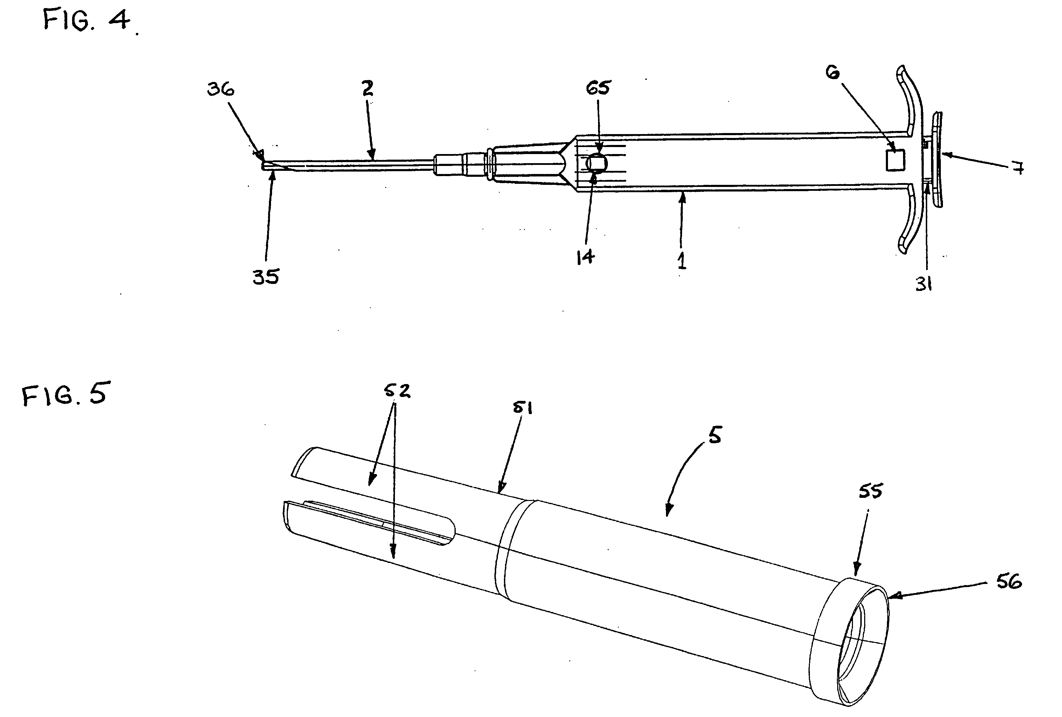

Finally, as shown in Figure 4, the user depresses the handle 7 of the plunger segment 31, which in turn drives the plunger rod 35 through the hollow needle 2, because the plunger segment 31 and plunger rod 35 are now rigidly coupled together. As the plunger 3 is depressed, the catch arrangement 6 between the plunger rod 35 and syringe barrel 1 is released. When the plunger 3 is fully depressed, a second catch arrangement 65 between the plunger rod 35 and the syringe barrel 1 engages, to lock the plunger 3 in its fully depressed. In this position, the plunger rod 35 extends the full length of the hollow needle 2 (and preferably a little past the end of the needle), to provide a blunt end 36 to the needle 2, to minimise the risk of needle stick injuries.

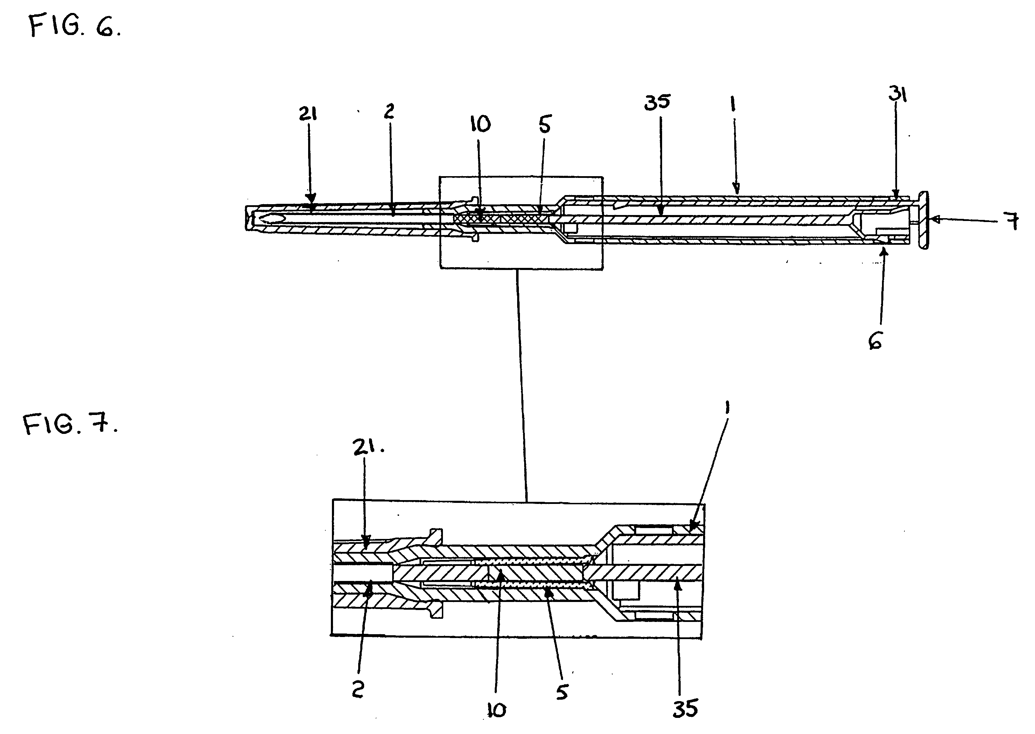

Referring to Figure 5, the medicament pellets are held in a specially designed clamp 5 to prevent them falling through the hollow needle 2. The clamp 5 comprises a first portion 51 adapted to fit inside the hollow needle 2 and having a plurality of flexible fingers 52, which can expand slightly to allow a medicament pellet to be inserted into the clamp 5, but thereafter relax to their original configuration to clamp the medicament pellet firmly therein. The clamp 5 also comprises a second portion 55, which is too large to fit through the hollow needle 2, but is shaped to align the first portion 51 of the clamp 5 within the hollow needle 2. Advantageously, the second portion 55 of the clamp presents a tapered orifice 56 to the inside of the syringe barrel 1, to assist with alignment of the plunger rod 35 with the pellets of medicament held in the clamp 5.

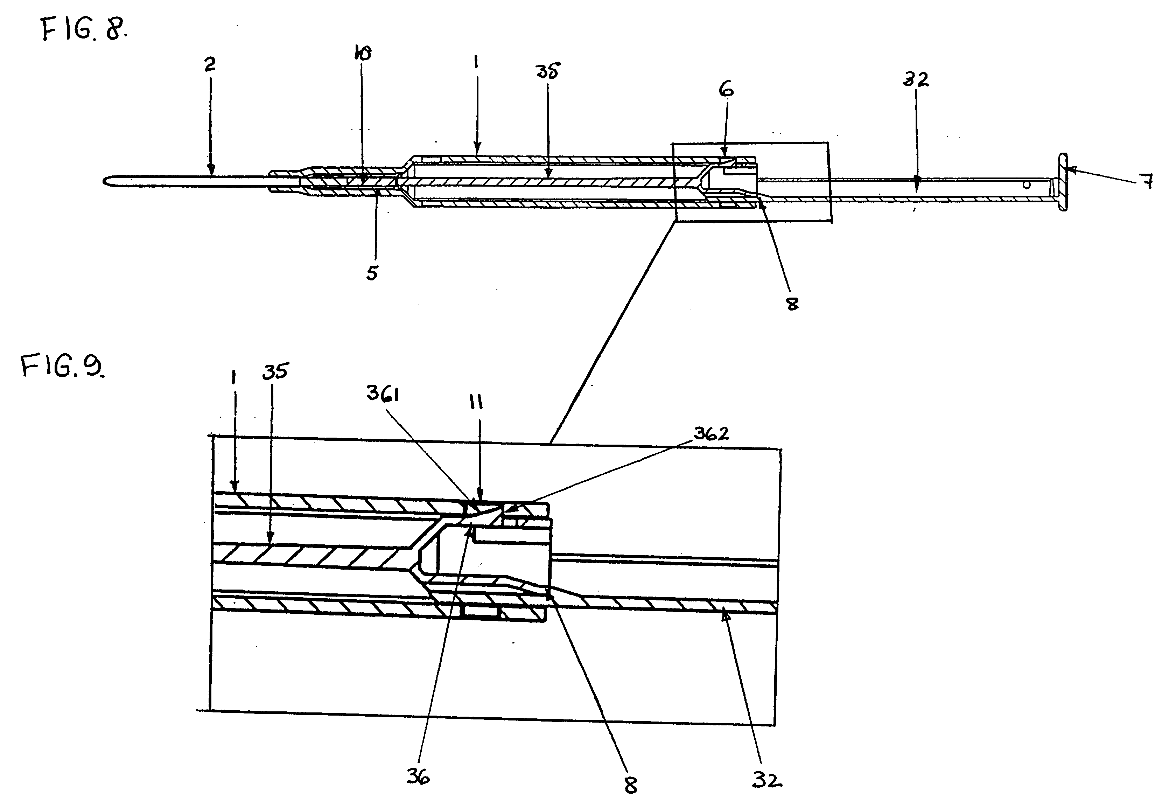

Now referring to figures 6 and 7, which show the arrangement of the telescopic plunger segment, rod, medicament clamp and pellets of medicament more clearly. As previously described, the syringe is provided to the end user with a cover 21 shielding the exposed hollow needle 2, primed with medicament. The medicament pellets 10 are inserted into the clamp 5 and then dropped into the hollow needle 2 through the open end of the barrel 1. The plunger segment 31 and plunger rod 35 are assembled together in a collapsed telescopic configuration, with the plunger rod 35 lying within the plunger segment 31 (as shown in Figure 6). The plunger assembly 3 is then also inserted into the open end of the syringe barrel 1. The free end of the plunger rod 35 is aligned with the medicament pellets 10 in the clamp 5 and the other end of the plunger rod 35 is attached to the syringe barrel by means of the catch arrangement 6.

Referring to Figures 8 and 9, the catch arrangement 6 comprises a resilient projection 36 extending from the plunger rod 35 (referenced in Figure 9), which engages in a slot 11 (referenced in Figure 9)in the syringe barrel 1. The resilient projection 36 has an engagement tooth, having a shallow sloping face 361 pointing towards the needle 2 and a steep or vertical flank 362, facing the exterior of the syringe barrel 1. In use, when the user pulls on the plunger handle 7 to extend the plunger 3, the steep or vertical flank of the engagement tooth engages with the wall of the slot 11 in the syringe barrel 1, to prevent the plunger rod 35 from being pulled out of the barrel 1. When the plunger segment 31 is fully extended, a further catch arrangement 8 between the plunger segment 31 and plunder rod 35 engages to form a rigid plunger 3.

The user then depresses the plunger 3, to eject the medicament tablet 10 from the end of hollow needle 2. The shallow sloping face 361 of the engagement tooth, slides across the edge of the slot 11, allowing the plunger 3 to be depressed. The user may either insert the hollow needle 2 into the injection site before of after extending the plunger 3. When the plunger 3 is then depressed, the plunger rod 35, pushes the medicament tablets 10 through the clamp 5, through the hollow needle 2 and into the injection site.

Preferably, the syringe barrel is manufactured from a transparent material to allow a user of the syringe to see the medicament tablets stored therein. This avoids confusion between unused, primed syringes and used, empty syringes. Alternatively, the syringe barrel may be provided with a transparent window to achieve the same effect.

For ease of assembly, the syringe barrel and plunger preferably has a polygonal shape to restrict the way they can be assembled together. Where the shape is rectangular (as shown in the drawings), the catches 6, 8 are preferably provided symmetrically on opposite sides of the barrel, to enable the plunger to be assembled in the barrel either way around.

## Claims

1. A syringe device comprising a barrel (1) for holding one or more tablets of medicament (10), a hollow needle (2) attached to the barrel (1) and a plunger (3) adapted to move within the barrel (1),
the plunger (3) having a plunger rod (35) adapted to move within the hollow needle (2) to drive a medicament tablet (10) through the needle, ***characterised in that***
the plunger (3) is composed of a plurality of segments, telescopically arranged one within the other,
the plunger rod (35) forming the innermost segment of the plunger (3), and
each segment having a catch (6), (8) to lock the segment in its extended position, wherein the catch (6) on the plunger rod (35) is adapted to prevent the plunger rod (35) being removed from the barrel (1) but allows the rod (35) to be depressed through the barrel (1) once the segments are fully extended and locked together.

2. A syringe device according to claim (1), wherein the catch (6) between the plunger rod (35) and the barrel (1) comprises a resilient projection (36) adapted to engage in a slot (11) and the projections has a smooth tapered face (361) in the direction of travel of the plunger rod (35) towards the free end of the needle (2) and a steep or vertical flank (362), which engages in the slot (11) when the plunger rod (35) is being pulled in a direction away from needle (2).

3. A syringe device according to any one of the preceding claims, wherein the plunger (3) comprises a plunger rod (35) and a body segment (31) telescopically arranged with the plunger rod (35) inside the body segment (31)

4. A syringe device according to claim 1 wherein another catch (65) is present at the needle end of the syringe barrel (1) to prevent the plunger rod (35) from being withdrawn through the needle, allowing to lock it in its fully depressed position.

5. A syringe device according to any of the preceding claims, further comprising a clamp (5) adapted to firmly hold one or more medicament tablets (10) within the syringe barrel (1) and prevent the tablet (10) from falling through the hollow needle (2).

6. A syringe device according to claim 5, wherein the clamp (5) is shaped to align the tablet with the hollow needle (2).

7. A syringe device according to claim 5 or claim 6, wherein the clamp (5) is shaped to direct the plunger rod (35) towards the medicament tablet (10).

8. A syringe device according to any one of claims 5 to 7, wherein the clamp (5) has a first portion (51) designed to slide into the hollow needle and a second portion (55) which cannot fit inside the needle but is used to align the clamp with the needle, and the clamp is weighted to ensure that it drops into the needle and the second portion self aligns in the syringe barrel.

9. A syringe device according to any one of the preceding claims, wherein the plunger rod (35) is of sufficient length to extend the full length of the needle (2) when fully depressed.

## Patentansprüche

1. Eine Spritzenvorrichtung umfassend einen Zylinder (1) zum Halten einer oder mehrerer Medikamenten-Tabletten (10) , eine an.dem Zylinder (1) befestigte Hohlnadel (2) und einen zu einer Bewegung in dem Zylinder (1) geeigneten Kolben (3),
wobei der Kolben (3) eine Kolbenstange (35) aufweist, die geeignet ist, sich in der Hohlnadel (2) zu bewegen, um eine Medikamenten-Tablette (10) durch die Nadel zu schieben, ***dadurch gekennzeichnet, dass*** der Kolben (3) aus mehreren Segmenten besteht, die teleskopfartig zueinander angeordnet sind,
wobei die Kolbenstange (35) das innerste Segment des Kolbens (3) bildet,
und jedes Segment eine Verriegelung (6), (8) aufweist, um das Segment in seiner verlängerten Stellung zu verriegeln, wobei die Verriegelung (6) auf der Kolbenstange (35) so angeordnet ist, dass vermieden wird, dass die Kolbenstange (35) aus dem Zylinder (1) entfernt wird, es jedoch der Stange (35) ermöglicht, durch den Zylinder (1) gedrückt zu werden, sobald die Segmente vollständig ausgefahren und miteinander verriegelt sind.

2. Eine Spritzenvorrichtung nach Anspruch 1, bei welcher die Verriegelung (6) zwischen der Kolbenstange (35) und dem Zylinder (1) eine zum Eingreifen in einen Schlitz (11) geeignete elastische Auskragung (36) umfasst und die Auskragung eine glatte geneigte Fläche (361) in Richtung der Bewegung der Kolbenstange (35) zum freien Ende der Nadel (2) hin und eine steile oder vertikale Flanke (362), die in den Schlitz (11) eingreift, wenn die Kolbenstange (35) in einer Richtung weg von der Nadel (2) gezogen wird, aufweist.

3. Eine Spritzenvorrichtung nach irgendeinem der vorangehenden Ansprüche, bei welcher der Kolben (3) eine Kolbenstange (35) und ein mit der sich im Rumpfsegment (31) befindenden Kolbenstange (35) ausziehbar angeordnetes Rumpfsegment (31) umfasst.

4. Eine Spritzenvorrichtung nach Anspruch 1, bei der eine weitere Verriegelung (65) am Nadelende des Spritzenzylinders (1) vorhanden ist, um zu vermeiden, dass die Kolbenstange (35) durch die Nadel herausgezogen wird, wodurch es ermöglicht wird, sie in ihrer vollständig heruntergedrückten Stellung zu verriegeln.

5. Eine Spritzenvorrichtung nach irgendeinem der vorangehenden Ansprüche, die außerdem eine Klemmhülse (5) umfasst, die dazu geeignet ist, eine bzw. mehrere Medikamenten-Tabletten (10) fest innerhalb des Spritzenzylinders (1) zu halten und zu verhindern, dass die Tablette (10) durch die Hohlnadel (2) fällt.

6. Eine Spritzenvorrichtung nach Anspruch 5, bei der die Klemmhülse (5) so geformt ist, dass die Tablette mit der Hohlnadel (2) in einer Flucht liegt.

7. Eine Spritzenvorrichtung nach Anspruch 5 oder 6, bei der die Klemmhülse (5) so ausgeformt ist, dass die Kolbenstange (35) zu der Medikamenten-Tablette (10) hin ausgerichtet ist.

8. Eine Spritzenvorrichtung nach irgendeinem der Ansprüche 5 bis 7, bei der die Klemmhülse (5) einen ersten Abschnitt (51), der so ausgeformt ist, dass er in die Hohlnadel gleitet, und einen zweiten Abschnitt (55) aufweist, der nicht in die Nadel passt, sondern verwendet wird, um die Klemmhülse mit der Nadel auszufluchten, und die Klemmhülse gewichtsbeschwert ist, um zu gewährleisten, dass sie in die Nadel fällt und der zweite Teil sich von selbst in dem Spritzenzylinder ausfluchtet.

9. Eine Spritzenvorrichtung nach irgendeinem der vorangehenden Ansprüche, bei der die Kolbenstange (35) lang genug ist um sich über die gesamte Länge der Nadel (2) zu erstrecken, wenn sie niedergedrückt ist.

## Revendications

1. Dispositif de seringue comprenant un cylindre (1) destiné à retenir un ou plusieurs comprimés de médicaments (10), une aiguille creuse (2) fixée au cylindre (1) et un piston (3) conçu pour se déplacer à l'intérieur du cylindre (1),
le piston (3) ayant une tige de piston (35) conçue pour se déplacer à l'intérieur de l'aiguille creuse (2) afin d'entraîner un comprimé de médicament (10) à travers l'aiguille, **caractérisé en ce que**
le piston (3) est composé d'une pluralité de segments, agencés de façon télescopique les uns dans les autres,
la tige de piston (35) formant le segment le plus à l'intérieur du piston (3), et
chaque segment ayant un élément d'arrêt (6), (8) pour bloquer le segment dans sa position déployée, l'élément d'arrêt (6) sur la tige de piston (35) étant conçu pour empêcher la tige de piston (35) d'être retirée du cylindre (1), mais permet à la tige (35) d'être enfoncée à travers la cylindre (1) une fois que les segments sont entièrement déployés et bloqués ensemble.

2. Dispositif de seringue selon la revendication (1), dans lequel l'élément d'arrêt (6) entre la tige de piston (35) et le cylindre (1) comprend une saillie élastique (36) conçue pour s'introduire dans une fente (11) et la saillie présente une face effilée lisse (361) dans le sens de déplacement de la tige du piston (35) vers l'extrémité libre de l'aiguille (2) et un flanc raide ou vertical (362) qui s'engage dans la fente (11) lorsque la tige du piston (35) est tirée dans une direction s'éloignant de l'aiguille (2).

3. Dispositif de seringue selon l'une quelconque des revendications précédentes, dans lequel le piston (3) comprend une tige de piston (35) et un segment de corps (31) agencé de façon télescopique avec la tige du piston (35) à l'intérieur du segment de corps (31).

4. Dispositif de seringue selon la revendication 1, dans lequel un autre élément d'arrêt (65) est présent à l'extrémité de l'aiguille du cylindre de la seringue (1) pour empêcher la tige du piston (35) d'être retirée à travers l'aiguille, en permettant de la bloquer dans sa position entièrement enfoncée.

5. Dispositif de seringue selon l'une quelconque des revendications précédentes, comprenant en outre un élément de serrage (5) adapté pour retenir fermement un ou plusieurs comprimés de médicaments (10) à l'intérieur du cylindre de la seringue (1) et empêcher le comprimé (10) de tomber à travers l'aiguille creuse (2).

6. Dispositif de seringue selon la revendication 5, dans lequel l'élément de serrage (5) est façonné pour aligner le comprimé avec l'aiguille creuse (2).

7. Dispositif de seringue selon la revendication 5 ou la revendication 6, dans lequel l'élément de serrage (5) est façonné pour orienter la tige du piston (35) en direction du comprimé de médicament (10).

8. Dispositif de seringue selon l'une quelconque des revendications 5 à 7, dans lequel l'élément de serrage (5) présente une première partie (51) conçue pour coulisser dans l'aiguille creuse et une seconde partie (55) qui ne peut pas s'emboîter à l'intérieur de l'aiguille mais est utilisée pour aligner l'élément de serrage avec l'aiguille, et l'élément de serrage est alourdi pour s'assurer qu'il entre dans l'aiguille et que sa seconde partie s'aligne automatiquement dans le cylindre de la seringue.

9. Dispositif de seringue selon l'une quelconque des revendications précédentes, dans lequel la tige de piston (35) est d'une longueur suffisante pour s'étendre sur la longueur totale de l'aiguille (2) lorsqu'elle est entièrement enfoncée.
